(19) **Europäisches Patentamt / European Patent Office / Office européen des brevets**

(11) **EP 4 062 902 A1**

(12) **EUROPEAN PATENT APPLICATION**
published in accordance with Art. 153(4) EPC

(43) Date of publication:
**28.09.2022 Bulletin 2022/39**

(21) Application number: **20907372.5**

(22) Date of filing: **14.12.2020**

(51) International Patent Classification (IPC):
**A61K 8/98** (2006.01)        **A61Q 19/08** (2006.01)
**A23L 33/10** (2016.01)        **A61K 35/20** (2006.01)
**A61P 17/00** (2006.01)

(52) Cooperative Patent Classification (CPC):
**A23L 33/10; A61K 8/98; A61K 35/20; A61P 17/00; A61Q 19/08**

(86) International application number:
**PCT/KR2020/018279**

(87) International publication number:
**WO 2021/132970 (01.07.2021 Gazette 2021/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.12.2019 KR 20190176590**

(71) Applicant: **UNIVERSITY-INDUSTRY COOPERATION GROUP OF KYUNG HEE UNIVERSITY**
**Yongin-si, Gyeonggi-do 17104 (KR)**

(72) Inventors:
- **KIM, Sang Hoon**
  **Seoul 07002 (KR)**
- **BAE, Inseon**
  **Seoul 02447 (KR)**

(74) Representative: **Isarpatent**
**Patent- und Rechtsanwälte Barth**
**Charles Hassa Peckmann & Partner mbB**
**Friedrichstrasse 31**
**80801 München (DE)**

(54) **COMPOSITION FOR SKIN ELASTICITY ENHANCEMENT AND WRINKLE IMPROVEMENT COMPRISING MILK EXOSOMES**

(57)    The present invention relates to a composition for enhancing skin elasticity and/or ameliorating wrinkles, including milk exosomes, and a method for treating wrinkles by administering milk exosomes.

【Fig. 5】

**Description**

**TECHNICAL FIELD**

The present invention is related to a composition for enhancing skin elasticity and/or alleviating wrinkles, including milk exosomes, and a method for treating wrinkles by administering milk exosomes.

**BACKGROUND ART**

The skin is a membrane that covers the outside of the body and serves to protect and regulate internal organs by performing a variety of physiological functions that protect the body from environmental factors such as various external stimuli, obstacles, and dryness.

However, as the age of the skin increases, the physiological functions of an organism deteriorate, so the secretion of various hormones that regulate metabolism is reduced and the functions of immune cells and the activity of cells deteriorate. Further, repeated exposure to light or heat alters the appearance or function of the skin, which reduces collagen synthesis and causes moisture loss in the skin, and damages the skin by promoting a skin aging phenomenon while reducing skin elasticity. One of the representative phenomena of skin aging is the formation of wrinkles.

To maintain healthier and more beautiful skin by preventing such skin aging and damage, efforts have been continuously made to maintain the unique function of the skin by additionally using bioactive materials obtained from various animals, plants and microorganisms in cosmetics. Additionally, one can maintain skin health by activating skin cells to promote skin metabolism, and accordingly, cosmetics, skin external preparations, and the like have been developed.

However, these materials have safety issues, such as causing irritation, erythema, redness, and the like, when applied to the skin and thus have limited usage, or they have little effect on the skin and hence make it difficult to expect a substantial skin function improving effect.

Therefore, there is a need for research and development of a material having excellent skin elasticity enhancement and/or wrinkle improvement effects.

**DESCRIPTION OF EMBODIMENTS**

**TECHNICAL PROBLEM**

The technical problem to be solved by the present invention is to provide a composition for enhancing skin elasticity and/or ameliorating wrinkles, including milk exosomes.

Another technical problem that the present intervention addresses is to provide a cosmetic composition for enhancing skin elasticity and/or ameliorating wrinkles, including milk exosomes.

Another technical problem to be solved by the present invention is to provide a food composition for enhancing skin elasticity and/or ameliorating wrinkles, including milk exosomes.

Another technical problem that the present intervention addresses is to provide a pharmaceutical composition for enhancing skin elasticity and/or preventing or treating wrinkles, including milk exosomes.

Finally, another technical problem to be solved by the present invention is to provide a method for treating wrinkles, the method including administering milk exosomes to an individual.

**TECHNICAL SOLUTION**

An aspect of the present invention to achieve the aforementioned objects related to a composition for enhancing skin elasticity and ameliorating wrinkles, including milk exosomes.

In the present invention, "milk exosome" refers to a vesicle of a lipid bilayer derived from milk. The exosomes contain unique proteins, nucleic acids and the like derived from milk, and are biological nanoparticles with a size of 30 to 200 nm, and information consisting of DNA, RNA, peptides, and the like is contained therein. Exosomes affect the microenvironment around cells by safely transporting internal materials from lyases in body fluids, and the like to transmit information to adjacent cells or distant cells. In particular, milk-derived milk exosomes may be used as food because not only a large amount of exosomes are extracted, but also there is an advantage in that they can be stored in a stable state for a long time.

Meanwhile, although exosomes in the related field have been often used as biomarkers or carriers, recently, various small RNAs are present in milk exosomes that are widely used in the manufacture of various dairy products as well as breast milk, and it has been revealed that the genetic materials are involved in immune function or an osteoclast generation process and are useful for the treatment and amelioration of osteoporosis (Mizoguchi et al., 2010). Accordingly, the present inventors have conducted research on milk exosomes, and clarified for the first time that the milk exosomes are involved in gene expression regulation related to skin elasticity enhancement and wrinkle amelioration, collagen synthesis, inhibition of collagenase and elastase activity, and the like.

Accordingly, in the present invention, exosomes were obtained by centrifugation from cow-derived milk, and the milk exosomes are intended to be used for skin elasticity enhancement and wrinkle amelioration. Specifically, the exosome may be derived from cows.

In the present invention, "enhancement of skin elasticity" refers to ameliorating wrinkles caused by the reduction in skin elasticity due to skin aging. That is, when skin elasticity is reduced, the movement of folding and unfolding the muscles under the epidermis is repeated due to the reduction and degeneration of collagen fibers in the skin, thereby causing skin wrinkles. Accordingly, enhancement of skin elasticity includes both preventing and suppressing the generation of wrinkles by improving skin elasticity that has deteriorated.

In the present invention, "skin wrinkles" refers to fine lines caused by skin deterioration, and may be caused by genetic causes, a reduction in collagen and elastin present in the skin dermis, an external environment, and the like. Accordingly, the "skin wrinkle amelioration" refers to suppressing or inhibiting the generation of wrinkles on the skin, or alleviating the already generated wrinkles.

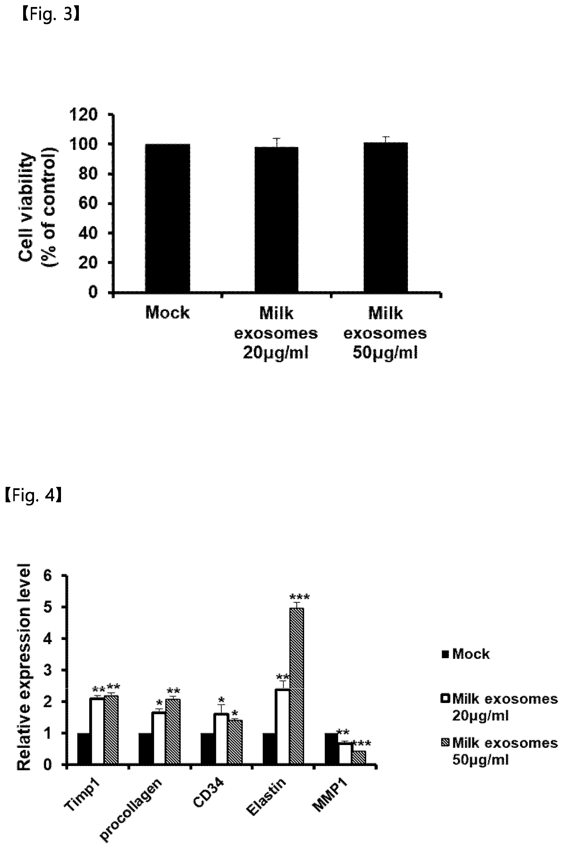

In an exemplary embodiment of the present invention, as a result of confirming the expression pattern of skin elasticity and/or wrinkle-related genes, it was confirmed that the expression level of an MMP-1 gene was decreased, while the genes expression levels of TIMP1, procollagen, CD34, and elastin (elastin) were all increased (FIG. 4). The aforementioned results suggest that milk exosomes can be utilized as a composition for enhancing skin elasticity and ameliorating wrinkles by regulating the expression of skin elasticity enhancement and wrinkle-related genes.

The milk exosomes may inhibit elastase activity and collagenase activity.

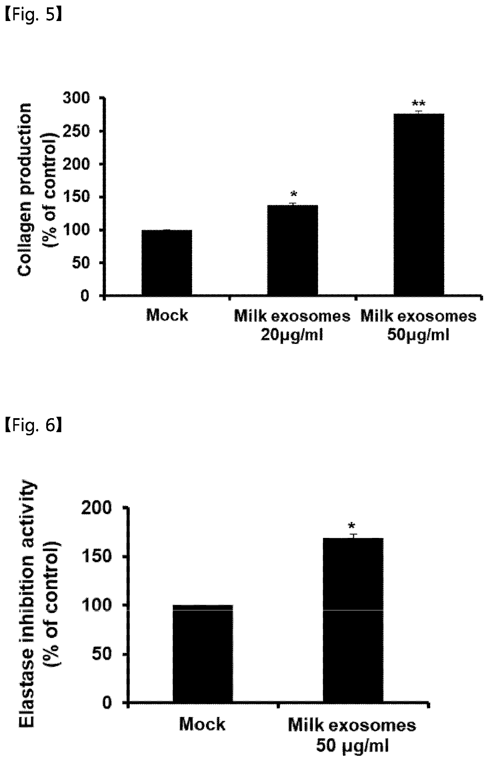

The elastase is one of the leukocyte granulation enzymes that degrade elastin in the body by participating in the degradation of elastin, which is a matrix protein that maintains skin elasticity in the dermis. When the activity of the enzyme is increased, it is a direct cause of destruction of skin tissue, and thus elastase is known to cause wrinkles and reduced elasticity of the skin. In an exemplary embodiment of the present invention, it was confirmed that when the milk exosomes were treated, the activity of elastase was significantly inhibited (FIG. 6).

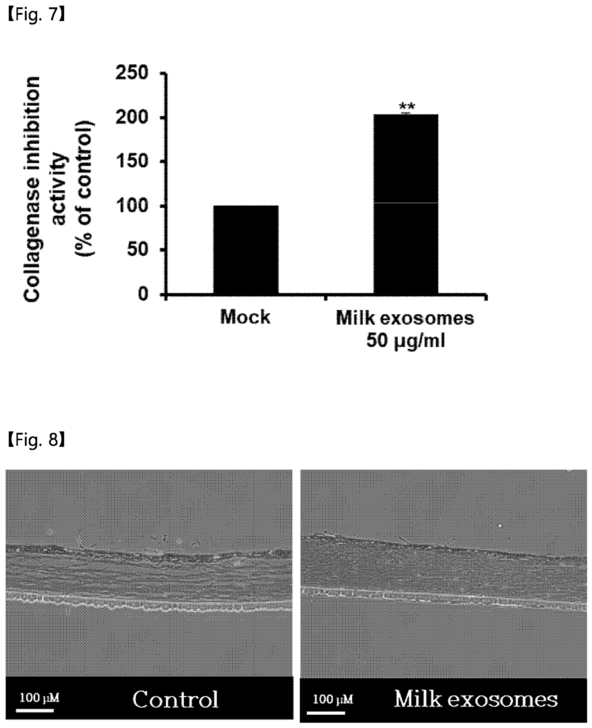

The collagenase is one of the enzymes that degrade collagen, and is also known as matrix metalloproteinase I (MMP-1) that degrades collagen type I. In an exemplary embodiment of the present invention, as a result of measuring the collagenase activity inhibitory effect by treating the milk exosomes, it was confirmed that the collagenase activity was remarkably inhibited compared to a milk exosome untreated group (FIG. 7).

Accordingly, it was confirmed that milk exosomes can be utilized for skin elasticity enhancement and wrinkle amelioration purposes by inhibiting or suppressing the activity of elastase and collagenase enzymes, which are known to be a direct cause of skin elasticity deterioration and wrinkle formation.

In addition, specifically, the composition may further include an aging amelioration use.

In the present invention, aging is divided into intrinsic aging and extrinsic aging; intrinsic aging is a phenomenon in which physical functions naturally decline over time, and extrinsic aging includes both photoaging caused by sunlight and aging caused by other factors such as smoking, drinking and stress.

The aging may be specifically photoaging.

The photoaging is caused by cell aging and apoptosis by DNA damage and a change in gene expression by ultraviolet rays affecting the signal transduction system in skin cells. When skin cells are continuously exposed to UV rays, aging is caused while active oxygen is increased in fibroblasts of the dermal layer, the expression of cytokine transforming growth factor-beta (TGF-$\beta$) is reduced, and the expression of activator protein 1 (AP-1) is activated.

For example, TGF-$\beta$ is a cytokine that promotes collagen synthesis, and when the expression of TGF-$\beta$ is reduced by UV rays, collagen synthesis is inhibited, whereas AP-1 promotes collagen degradation by regulating MMP to promote collagen degradation.

In an exemplary embodiment of the present invention, it was confirmed that milk exosomes could increase the amount of collagen biosynthesis inhibited by aging (FIG. 5) and prevent collagen degradation by suppressing the activity of collagenase and elastase (FIG. 7).

In an exemplary embodiment of the present invention, as a result of treatment with milk exosomes after inducing photoaging by treatment with ultraviolet UVB, it was confirmed that, compared to the control which was not treated with milk exosomes, there was almost no decrease in the amount of collagen fibers, the fiber density was dense, and the arrangement was regular (FIG. 8).

The aforementioned results suggest that milk exosomes have excellent effects not only on aging caused by deterioration of skin elasticity due to deterioration of physical functions, but also on prevention and amelioration of extrinsic aging by ultraviolet rays.

Another object of the present invention relates to a cosmetic composition for enhancing skin elasticity and ameliorating wrinkles, including milk exosomes.

The description on the 'milk exosomes', 'skin elasticity enhancement,' and 'wrinkle amelioration' is the same as described above.

Specifically, the milk exosomes may be derived from cows.

In an exemplary embodiment of the present invention, as a result of treating human dermal fibroblasts with cow-derived milk exosomes, it was confirmed that there was almost no cytotoxicity (FIG. 3), and thus, verifying that milk exosomes could be utilized in cosmetic compositions.

In an exemplary embodiment of the present invention, it was confirmed that milk exosomes regulated the expression of skin elasticity and wrinkle-related genes (FIG. 4.), increase the amount of collagen synthesis (FIG. 5), and in particular, have excellent effects on the inhibition of the enzyme activity of elastase and collagenase (FIGS. 6 and 7), and thus milk exosomes could indeed be utilized as a cosmetic composition for enhancing skin elasticity and ameliorating wrinkles.

Furthermore, the cosmetic composition may further include an aging amelioration use. Specifically, the aging may be photoaging.

In an exemplary embodiment of the present invention, it was confirmed that milk exosomes showed little decrease in collagen even in the case of treatment with ultraviolet UVB, and the fiber density was dense and the arrangement was regular (FIG. 8). Thus, through the above results, milk exosomes could be utilized as a cosmetic composition for ameliorating aging caused by ultraviolet rays.

The cosmetic composition of the present invention may be prepared as a formulation selected from the group consisting of a solution, an external ointment, a cream, a foam, a nourishing lotion, a soft lotion, a pack, soft water, an emulsion, a makeup base, an essence, soap, a liquid detergent, a bath agent, a sunscreen cream, a sun oil, a suspension, an emulsion liquid, a paste, a gel, a lotion, a powder, soap, a surfactant-containing cleanser, an oil, a powder foundation, an emulsion foundation, a wax foundation, a patch and a spray, but is not limited thereto.

The cosmetic composition of the present invention may further include one or more cosmetically acceptable carriers to be blended in general skin cosmetics, and as a common ingredient, for example, an oil ingredient, water, a surfactant, a humectant, a lower alcohol, a thickener, a chelating agent, a colorant, a preservative, a fragrance, and the like may be appropriately blended, but are not limited thereto.

The cosmetically acceptable carrier included in the cosmetic composition of the present invention varies depending on the formulation of the cosmetic composition.

When the formulation of the present invention is an ointment, a paste, a cream or a gel, animal oil, vegetable oil, wax, paraffin, starch, traganth, a cellulose derivative, polyethylene glycol, silicone, bentonite, silica, talc, zinc oxide, and the like may be used as a carrier ingredient, but the carrier ingredient is not limited thereto. These may be used alone or in a mixture of two or more thereof.

When the formulation of the present invention is a powder or a spray, lactose, talc, silica, aluminum hydroxide, calcium silicate, a polyamide powder, and the like may be used as the carrier ingredient, and in particular, when the formulation is a spray, the formulation may additionally include a propellent such as chlorofluorohydrocarbon, propane/butane or dimethyl ether, but is not limited thereto. These may be used alone or in a mixture of two or more thereof.

When the formulation of the present invention is a solution or emulsion, a solvent, solubilizer, or emulsifier may be used as the carrier ingredient, for example, water, ethanol, isopropanol, ethyl carbonate, ethyl acetate, benzyl alcohol, benzyl benzoate, propylene glycol, 1,3-butyl glycol oil and the like may be used, and in particular, cottonseed oil, peanut oil, corn germ oil, olive oil, castor oil and sesame oil, glycerol aliphatic esters, fatty acid esters of polyethylene glycol or sorbitan may be used, but the carrier ingredient is not limited thereto. These may be used alone or in a mixture of two or more thereof.

When the formulation of the present invention is a suspension, a liquid diluent such as water, ethanol or propylene glycol, a suspending agent such as ethoxylated isostearyl alcohol, polyoxyethylene sorbitol ester and polyoxyethylene sorbitan ester, microcrystalline cellulose, aluminum metahydroxide, bentonite, agar, traganth, or the like may be used as the carrier ingredient, but the carrier ingredient is not limited thereto. These may be used alone or in a mixture of two or more thereof.

Further, the composition of the present invention may be used by a transdermal administration method, such as directly applied to the skin or sprayed, and the administration route of the composition of the present invention may be administered through any general route as long as it can reach a target tissue.

The usage amount of the composition of the present invention may be appropriately adjusted according to individual differences or formulations such as age and severity of a lesion, and may be used for one week to several months by applying a suitable amount of the composition to the skin once to several times a day.

Another aspect of the present invention relates to a food composition for enhancing skin elasticity and ameliorating wrinkles, including milk exosomes.

The description on the 'milk exosomes', 'skin elasticity enhancement' and 'wrinkle amelioration' is the same as described above.

The type of food is not particularly limited. Foods to which the milk exosome of the present invention may be added include sausage, meat, bread, chocolate, snacks, candy, confectioneries, ramen, pizza, other noodles, gum, dairy products including ice cream, various soups, beverages, tea, drinks, alcoholic beverages, and vitamin complexes. When formulated as a beverage, the liquid component added in addition to the milk exosome of the present invention is not limited thereto, but may contain various flavoring agents or natural carbohydrates as additional components as in a typical beverage. The above-described natural carbohydrates may be monosaccharides (for example, glucose, fructose, and the like), disaccharides (for example, maltose, sucrose, and the like) and polysaccharides (for example, typical sugars such as dextrin and cyclodextrin), and a sugar alcohol such as xylitol, sorbitol and erythritol.

The type of food may specifically be a health functional food. More specifically, the type of food may be a health functional food for enhancing skin elasticity and ameliorating wrinkles.

The health functional food may contain various nutritional supplements, vitamins, minerals (electrolytes), flavoring agents such as synthetic flavoring agents and natural flavoring agents, colorants and enhancers (cheese, chocolate, and the like), pectic acid and salts thereof, organic acids, protective colloid thickeners, pH adjusting agents, stabilizers, preservatives, glycerin, alcohol, carbonating agents used in a carbonated beverage, and the like. In addition, the health functional food of the present invention may contain the pulp for the preparation of fruit and vegetable beverages. These components may be used alone or in combination, and the proportion of these additives is generally selected in a range of 0.001 to 50 parts by weight based on the total weight of the composition.

The health functional food is a food that emphasizes the bioregulatory function of food, and is a food with added value to act for and express a specific purpose using a physical, biochemical, and bioengineering method. The ingredients of these health functional foods are designed and processed to sufficiently exert the body control functions related to the biological defense, regulation of body rhythm, and prevention and recovery of diseases for organisms and may contain food supplement additives, sweeteners or functional raw materials that are acceptable as food.

When the milk exosome of the present invention is used as health functional foods (or health functional beverage additives), the milk exosome is added as it is or used with other foods or food ingredients, and may be appropriately used according to a typical method. The mixing amount of the milk exosome may be suitably determined according to the purpose of their use (prevention, health or improvement, therapeutic treatment).

Still another aspect of the present invention relates to a pharmaceutical composition for enhancing skin elasticity and preventing or treating wrinkles, including milk exosomes.

In the present invention, "improvement of skin elasticity" refers to ameliorating wrinkles caused by the reduction in skin elasticity due to skin aging. That is, when skin elasticity is reduced, the movement of folding and unfolding the muscles under the epidermis is repeated due to the reduction and degeneration of collagen fibers in the skin, thereby causing skin wrinkles. Accordingly, improvement of skin elasticity includes both preventing and suppressing the generation of wrinkles by improving skin elasticity that has deteriorated.

In the present invention, the "skin wrinkle amelioration" refers to suppressing or inhibiting the generation of wrinkles on the skin, or alleviating the already generated wrinkles.

Specifically, the milk exosomes may be derived from cows.

In an exemplary embodiment of the present invention, it was confirmed that milk exosomes had little cytotoxicity against dermal fibroblasts (FIG. 3).

In an exemplary embodiment of the present invention, milk exosomes regulate the expression of genes related to elasticity deterioration and wrinkle formation and/or inhibition (FIG. 4), promote collagen synthesis (FIG. 5), and in particular, it was directly confirmed that milk exosomes inhibited elastase and collagenase activity (FIGS. 6 and 7).

Furthermore, specifically, the composition may further include an anti-aging or therapeutic use.

In an exemplary embodiment of the present invention, as a result of treatment with milk exosomes after inducing photoaging by treatment with ultraviolet UVB, it was confirmed that, compared to a group which was not treated with milk exosomes, there was almost no decrease in the amount of collagen fibers, the fiber density was dense, and the arrangement was regular (FIG. 8).

From the aforementioned results, it can be seen that since the pharmaceutical composition including the milk exosome of the present invention inhibits gene expression and enzyme activity that interfere with collagen synthesis while inducing collagen synthesis, the pharmaceutical composition may be used for skin elasticity enhancement and wrinkle amelioration, and furthermore, the pharmaceutical composition may be used for the prevention or treatment of aging caused by ultraviolet rays, and the like.

For administration, the pharmaceutical composition of the present invention may include a pharmaceutically acceptable carrier, excipient or diluent in addition to the milk exosome of the present invention. Examples of the carrier, the excipient or the diluent include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

Further, the pharmaceutical composition of the present invention can be applied in any dosage form, and more specifically, it may be a formulation for parenteral use. The formulation for parenteral use may be in the form of an injection, an application, a spray, such as an aerosol. More specifically, it may be in the form of an injection.

Examples of a preparation for parenteral administration include an aqueous sterile solution, a non-aqueous solvent, a suspension, an emulsion, a freeze-dried preparation, and a suppository. As the non-aqueous solvent and the suspension, it is possible to use propylene glycol, polyethylene glycol, a vegetable oil such as olive oil, an injectable ester such as ethyl oleate, and the like.

Yet another aspect of the present invention relates to a method for enhancing skin elasticity and treating wrinkles, the method including administering a pharmaceutical composition including milk exosomes to an individual in need of treatment in a pharmaceutically effective amount. The description on the 'skin elasticity' and 'wrinkle' is the same as described above. Specifically, it may relate to a method for treating wrinkles by administering a pharmaceutical composition including milk exosomes.

In addition, the treatment method may further include aging treatment. 'Aging' is the same as described above.

In the present invention, "pharmaceutically effective amount" means an amount sufficient to treat diseases at a reasonable benefit/risk ratio applicable to medical treatment, and an effective dosage level may be determined according to factors including the sexually transmitted disease, age, and type of disease of a patient, the severity of disease, the activity of drugs, sensitivity to drugs, administration time, administration route, excretion rate, treatment period, and simultaneously used drugs, and other factors well known in the medical field.

The pharmaceutical composition of the present invention may be administered as an individual therapeutic agent or may be administered in combination with other therapeutic agents, and may be administered sequentially or simultaneously with therapeutic agents in the related field. In addition, the pharmaceutical composition of the present invention may be administered in a single dose or multiple doses. It is important to administer the composition in a minimum amount that can obtain the maximum effect without any side effects, in consideration of all the aforementioned factors, and this amount may be easily determined by the person skilled in the art.

The term "individual" of the present invention includes animals or humans having a skin pigmentation disease whose symptoms can be ameliorated by administration of the pharmaceutical composition according to the present invention. By administering the therapeutic composition according to the present invention to an individual, skin pigmentation diseases may be effectively prevented and treated.

As used herein, the term "administration" refers to introduction of a predetermined material to a human or animal by any appropriate method, and for the route of administration of the therapeutic composition according to the present invention, the therapeutic composition of the present invention may be orally or parenterally administered via any general route, which may reach a target tissue. Furthermore, the therapeutic composition according to the present invention may be administered by any device which may allow an active ingredient to move to a target cell.

A preferred dosage of the pharmaceutical composition according to the present invention varies depending on the condition and body weight of a patient, the degree of a disease, the form of drug, the administration route, and the duration, but may be appropriately selected by a person skilled in the art.

**ADVANTAGEOUS EFFECTS OF INVENTION**

The composition including the milk exosome of the present invention is nontoxic, and thus has excellent biocompatibility, and has excellent effects of inhibiting elastase and collagenase enzyme activity and synthesizing collagen, so the composition can be used in various fields to improve skin elasticity and ameliorat wrinkles.

The effect of the present invention is not limited to the aforementioned effects, and it should be understood to include all possible effects deduced from the configuration of the invention described in the detailed description or the claims of the present invention.

**BRIEF DESCRIPTION OF THE DRAWINGS**

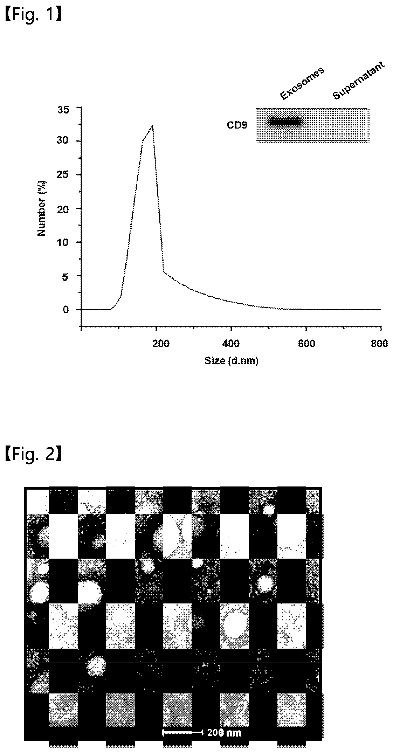

FIG. 1 shows the results of confirming the size of milk exosomes and exosome markers.
FIG. 2 shows the results of confirming the morphology and size of milk exosomes using an electron microscope.
FIG. 3 shows the results of confirming the toxicity of milk exosomes through cell viability.
FIG. 4 shows the mRNA expression levels of genes involved in skin elasticity and wrinkle formation/suppression of milk exosomes.
FIG. 5 shows the results of measuring the production amount of collagen of milk exosomes.
FIG. 6 shows the results of measuring elastase inhibitory activity by treating human dermal fibroblasts with milk exosomes.
FIG. 7 shows the results of measuring collagenase inhibitory activity by treating human dermal fibroblasts with milk exosomes.
FIG. 8 shows the results of measuring the effect of milk exosomes on amelioration of the photoaging phenomenon.

## DETAILED DESCRIPTION OF EXEMPLARY EMBODIMENTS

Hereinafter, the present invention will be described in detail through the Examples. However, the following Examples are only for exemplifying the present invention, and the present invention is not limited by the following Examples.

### Example 1. Method for preparing milk exosomes

Exosomes were extracted from cow-derived milk using a centrifuge. More specifically, milk was aliquoted into tubes, and the supernatant was collected by centrifugation at 2000 g and 10,000 g for 10 minutes, respectively. The collected supernatant was filtered through 0.45 $\mu$m and 0.2 $\mu$m filters, and then mixed with phosphate buffered saline (PBS). Thereafter, the Exoquick exosome precipitation solution (System Biosciences) was mixed with PBS, the resulting mixture was added thereto, and the resultant was allowed to stand for 30 minutes, and centrifuged again at 1,500 g for 30 minutes or 10,000 g for 10 minutes. The supernatant was removed, and an exosome pellet was dissolved in PBS and used for subsequent experiments.

### Experimental Example 1: Confirmation of characteristics of milk exosomes

#### 1-1. Measurement of size of milk exosomes

In order to confirm the size of the milk exosomes, the size of the exosomes was measured through dynamic light scattering (DLS), and it was confirmed that the size of the milk exosomes was 80 to 190 nm (FIG. 1).

#### 1-2. Confirmation of CD9 protein expression

The expression of CD9, which is an exosome marker, was confirmed using western blot, and as illustrated in FIG. 1, CD9 protein, which is an exosome marker, was clearly observed in exosomes, and it was confirmed that CD9 was not expressed in the supernatant other than the exosomes.

Proteins were isolated from milk exosomes using a 10% SDS polyacrylamide gel. After the isolated protein was transferred to a nitrocellulose membrane (GE Healthcare), it was reacted with a 5% skim milk powder for 1 hour to prevent the non-specific binding of an antibody. A CD9 (Abcam) primary antibody was diluted at a ratio of 1:1000 and bound to the membrane at 4°C for 12 to 18 hours. Thereafter, it was combined with an HRP-tagged anti-rabbit antibody and reacted at room temperature for 30 minutes. Protein bands were observed using an ECL kit (Santa Cruz Biotechnology).

#### 1-3. Confirmation of morphology and size of exosomes

The morphology and size of the milk exosomes extracted in Example 1 were observed using an electron microscope. As a result, as illustrated in FIG. 2, it was confirmed that the exosomes were present in a spherical shape within a size of 200 nm in diameter.

### Experimental Example 2. Confirmation of cytotoxicity of milk exosomes

#### 2-1. Human dermal fibroblast culture

Normal human dermal fibroblasts (NHDF), which are human dermal fibroblasts, were purchased from Lonza (New Jersey, USA) and used, and cultured in an incubator at 37°C and 5% $CO_2$ using an FGM-2 medium (Lonza, New Jersey, USA) supplemented with 2% fetal bovine serum (FBS), 0.1% insulin, 0.1% recombinant human fibroblast growth factor-$\beta$, and 0.1% GA-1000.

#### 2-2. Confirmation of cytotoxicity

In order to determine whether the milk exosomes of the present invention are toxic, cell viability was measured by treating NHDF cells with 20 $\mu$g/ml and 50 $\mu$g/ml of milk exosomes for 24 hours, respectively. A group not treated with milk exosomes was used as a control (Mock).

NHDF cells were aliquoted at a volume of $5 \times 10^4$ cells/ml in a 96-well plate and cultured in an incubator under conditions of 37°C and 5% $CO_2$ for 24 hours. The cultured cells were treated with milk exosomes and cultured for 24 hours, and then the effect on cell viability was measured using an EZ-Cytox cell viability assay kit (Daeil Lab Service) according to the manufacturer's method.

As a result, as illustrated in FIG. 3, there was no change in viability in both the control and the group treated with milk exosomes, confirming that the milk exosome of the present invention was not toxic.

## Experimental Example 3. Confirmation of effect of milk exosomes on skin elasticity improvement and wrinkle amelioration

### 3-1. Confirmation of expression patterns of skin elasticity and wrinkle-related genes

In order to investigate the expression patterns of skin elasticity and wrinkle-related genes according to treatment with milk exosomes, NHDF cells were treated with milk exosomes, and expression patterns of Timp1, procollagen, CD34, elastin and MMP-1 were confirmed by qRT-PCR.

NHDF cells were treated with milk exosomes and the cells were collected after 24 hours. Total RNA was isolated by reacting the collected cells with Tri reagent (Bioline). In order to perform a reverse transcription reaction, dNTP, M-MLV reverse-transcriptase (Promega), and the like were added to 1 $\mu$g of RNA, and then reacted at 37°C for 1 hour to synthesize cDNA. A real-time polymerase chain reaction was performed using SYBR Green PCR Master mix (Bioline) to measure Timp1, procollagen, CD34, elastin and MMP-1 mRNA expression. PCR conditions for amplifying a specific gene were performed as follows. After reaction at 95°C for 10 minutes, the gene was amplified for 40 cycles with a cycle of 15 seconds at 95°C, 15 seconds at 60°C, and 15 seconds at 72°C. A target gene mRNA expression level was corrected with the expression level relative to the actin expression level. The primers used are as shown in the following Table 1.

[Table 1]

| SEQ ID NO | Primer | Sequence |
|---|---|---|
| 1 | Timp1 Forward | 5'- ACC ACC TTA TAC CAG CGT TAT GA-3' |
| 2 | Timp1 Reverse | 5'- GGT GTA GAC GAA CCG GAT GTC-3' |
| 3 | Procollagen Forward | 5'- GTG CGA TGA CGT GAT CTG TGA -3' |
| 4 | Procollagen Reverse | 5'- CGG TGG TTT CTT GGT CGG T -3' |
| 5 | CD34 Forward | 5'- CTA CAA CAC CTA GTA CCC TTG GA -3' |
| 6 | CD34 Reverse | 5'- GGT GAA CAC TGT GCT GAT TAC A- 3' |
| 7 | Elastin Forward | 5'- GCA GGA GTT AAG CCC AAG G -3' |
| 8 | Elastin Reverse | 5'- TGT AGG GCA GTC CAT AGC CA- 3' |
| 9 | MMP1 Forward | 5'- ATT GGA GCA GCAAGAGGC TGG GA -3' |
| 10 | MMP1 Reverse | 5'- TTC CAG GTA TTT CTG GAC TAA GT -3' |

As a result, as illustrated in FIG. 4, it was confirmed that the mRNA expression of the MMP-1 gene was suppressed in a concentration-dependent manner in NHDF cells treated with milk exosomes. Meanwhile, in the case of TIMP1, which is a gene involved in collagen and elastin protection, it was confirmed that the expression level was increased according to the treatment with milk exosomes, and it was also confirmed that the expression levels of procollagen, CD34, and elastin were also increased. The above results suggest that milk exosomes promote the expression of genes involved in wrinkle amelioration, while there is an excellent effect on skin wrinkle amelioration by regulating the expression level of collagendegrading enzyme-related genes.

### 3-2. Confirmation of production amount of type I collagen

In order to confirm the collagen synthesis promoting effect of milk exosomes, the amount of collagen synthesis was confirmed after NHDF cells were treated with the milk exosomes. The production amount of collagen was measured by treating NHDF cells with milk exosomes at 20 $\mu$g/ml and 50 $\mu$g/ml, respectively, and a group not treated with milk exosomes was used as a control (Mock).

Specifically, NHDF cells were aliquoted at $1.5 \times 10^5$ cells/well, pre-cultured for 24 hours, then treated with milk exosomes at 20 $\mu$g/ml and 50 $\mu$g/ml, and cultured for 48 hours. Thereafter, a culture medium was centrifuged at 3000 rpm for 10 minutes, and then a Sircol collagen assay kit (Bioclolor, UK) was used. The obtained supernatant was treated with the isolation & concentration reagent provided in the kit and maintained at 4°C for 16 hours or more, and then centrifuged at 12,000 rpm for 10 minutes to concentrate collagen. After the supernatant was removed, 1 ml of the provided sircol dye reagent was added to the pellet and the pellet was maintained for 30 minutes. After centrifugation at 12,000 rpm for 10 minutes, the pellet was washed with an acid-salt wash reagent, and then centrifuged again under the above conditions. After centrifugation, a dye adsorbed to collagen was dissolved by treatment with an alkali reagent, and absorbance was measured at a wavelength of 555 nm.

As a result, as illustrated in FIG. 5, it was confirmed that the amount of collagen synthesis was increased in the case of treatment with milk exosomes, and in particular, it was confirmed that the collagen synthesis promoting effect of the milk exosomes was significantly increased in a concentration-dependent manner.

3-3. Confirmation of elastase activity inhibitory effect

Elastase inhibitory activity was measured according to the Cannell method (R. J. Cannell, S. J. Kellam, A. M. Owsianka, & J. M. Walker, 1988). By using N-succinyl-(L-Ala)3-p-nitroanilide (Sigma Aldrich CO., USA) as a substrate, the production amount of p-nitroanilide produced from the substrate was measured at 415 nm at 37°C for 20 minutes.

Specifically, after 20 µl of milk exosomes were treated with 120 µl of 50 mM Tris-HCl buffer (pH 8.0), 10 µl of elastase (1 unit/ml) and 50 µl of N-succinyl-(L-Ala)3-p-nitroanilide as an elastase substrate were added thereto and the resulting mixture was reacted at 37°C for 20 minutes. After the reaction, absorbance was measured at 415 nm.

[Equation 1]

$$\text{Inhibition rate (\%)} = [1-(\text{absorbance of sample added group/absorbance of no addition group})] \times 100$$

As a result, as illustrated in FIG. 6, since it was confirmed that in the case of treatment with milk exosomes, the elastase activity inhibitory effect was remarkably increased compared to the control not treated with milk exosomes, it was confirmed that the elastase activity inhibitory effect was excellent.

3-4. Confirmation of collagenase activity inhibitory effect

Collagenase inhibitory activity was measured according to the method of Wunsch and Heindrich (E. Wunsch, & H. G. Heidrich, 1963).

Specifically, 4 mM $CaCl_2$ was added to a 0.1 M Tris-HCL buffer (pH 7.5), 0.15 ml of 0.2 mg/mL collagenase (Sigma Aldrich CO., USA) was added to a mixed solution of 0.25 ml of a substrate solution in which 0.3 mg/mL of 4-phenyl azobenzyloxycarbonyl-Pro-Leu-Gly-Pro-D-Arg (Sigma Aldrich CO., USA) was dissolved and 0.1 ml of milk exosomes, the resulting mixture was allowed to stand at room temperature for 20 minutes, then the reaction was stopped by adding 500 µL of 6% citric acid thereto, and then 2 mL of ethyl acetate was added thereto to measure the absorbance at 320 nm using a Vmax microplate spectrophotometer (Molecular Devices, Sunnyvale, CA, USA). The collagenase inhibitory activity was expressed as the absorbance inhibition rate of the groups with and without the sample solution.

[Equation 2]

$$\text{Inhibition rate (\%)} = [1-(\text{absorbance of sample added group/absorbance of no addition group})] \times 100$$

As a result, as illustrated in FIG. 7, since it was confirmed that the collagenase activity inhibitory effect was remarkably increased in the case of treatment with milk exosomes, it was confirmed there is an excellent effect on the inhibition of the collagenase enzyme activity by the milk exosomes.

**Experimental Example 4. Confirmation of aging amelioration effect of milk exosomes**

To investigate the effect of milk exosomes on a photoaging phenomenon caused by ultraviolet UVB, the amount and morphology of collagen fibers were observed by the Masson's trichrome staining method.

First, Neoderm-ED (Tego Science), which is an artificial skin composed of the epidermis and the dermis, was purchased and irradiated with 0.05 J/cm$^2$ of ultraviolet rays once a day for 8 days, and treated with 50 μg/ml of milk exosomes once every other day for 8 days.

As a result, as illustrated in FIG. 8, it was confirmed that in the case of the treatment with the milk exosomes, the density of collagen fibers was dense and the arrangement was regular, whereas in the control not treated with the milk exosomes, the collagen fibers were destroyed, the density was sparse, and the arrangement was irregular, and the amount of collagen fibers was reduced.

Through the above results, it was confirmed that the milk exosomes had an excellent effect on alleviating skin aging caused by ultraviolet rays.

The above-described description of the present invention is provided for illustrative purposes, and those skilled in the field to which the present invention pertains will understand that the present invention can be easily modified into other specific forms without changing the technical spirit or essential features of the present invention. Therefore, it should be understood that the above-described embodiments are only exemplary in all aspects and are not restrictive. For example, each constituent element which is described as a singular form may be implemented in a distributed form, and similarly, constituent elements which are described as being distributed may be implemented in a combined form.

The scope of the present invention is represented by the following claims, and it should be interpreted that the meaning and scope of the claims and all the changes or modified forms derived from the equivalent concepts thereof fall within the scope of the present invention.

## Claims

1. A composition for enhancing skin elasticity and ameliorating wrinkles, comprising milk exosomes.

2. The composition of claim 1, wherein the milk exosomes are derived from cows.

3. The composition of claim 1, further comprising an aging amelioration use.

4. The composition of claim 3, wherein the aging is photoaging.

5. A cosmetic composition for enhancing skin elasticity and ameliorating wrinkles, comprising milk exosomes.

6. The cosmetic composition of claim 5, wherein the milk exosomes are derived from cows.

7. The cosmetic composition of claim 5, further comprising an aging amelioration use.

8. The cosmetic composition of claim 7, wherein the aging is photoaging.

9. A food composition for enhancing skin elasticity and ameliorating wrinkles, comprising milk exosomes.

10. The food composition of claim 9, wherein the milk exosomes are derived from cows.

11. The food composition of claim 9, wherein the food composition is a health functional food.

12. A pharmaceutical composition for enhancing skin elasticity and preventing or treating wrinkles, comprising milk exosomes.

13. The pharmaceutical composition of claim 12, wherein the milk exosomes are derived from cows.

14. The pharmaceutical composition of claim 12, further comprising an aging prevention or treatment use.

15. A method for treating wrinkles, the method comprising administering milk exosomes to an individual.

16. The method of claim 15, wherein the milk exosomes are derived from cows.

17. The method of claim 15, further comprising aging treatment.

【Fig. 1】

【Fig. 2】

【Fig. 3】

【Fig. 4】

【Fig. 5】

【Fig. 6】

【Fig. 7】

【Fig. 8】

## INTERNATIONAL SEARCH REPORT

| International application No. |
| --- |
| **PCT/KR2020/018279** |

### A. CLASSIFICATION OF SUBJECT MATTER

**A61K 8/98**(2006.01)i; **A61Q 19/08**(2006.01)i; **A23L 33/10**(2016.01)i; **A61K 35/20**(2006.01)i; **A61P 17/00**(2006.01)i

According to International Patent Classification (IPC) or to both national classification and IPC

### B. FIELDS SEARCHED

Minimum documentation searched (classification system followed by classification symbols)

A61K 8/98(2006.01); A23C 7/04(2006.01); A23C 9/00(2006.01); A23L 11/00(2016.01); A61K 35/20(2006.01); A61K 45/00(2006.01); A61K 9/127(2006.01); A61K 9/133(2006.01); A61P 29/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 우유(milk), 엑소좀(exosome), 피부(skin), 탄력(elasticity), 주름(wrinkle), 광노화 (photoaging)

### C. DOCUMENTS CONSIDERED TO BE RELEVANT

| Category* | Citation of document, with indication, where appropriate, of the relevant passages | Relevant to claim No. |
| --- | --- | --- |
| Y | WO 2018-102397 A1 (PURETECH HEALTH LLC) 07 June 2018 (2018-06-07)<br>See paragraph [0606]; and claims 1 and 23-24. | 1-14 |
| Y | KR 10-2005-0024313 A (GROPEP LIMITED) 10 March 2005 (2005-03-10)<br>See paragraph [0044]; and claims 1, 12, 14 and 18-19. | 1-14 |
| A | WO 2018-170332 A1 (NUTECH VENTURES) 20 September 2018 (2018-09-20)<br>See entire document. | 1-14 |
| A | JP 2002-537346 A (BERNSTEIN, Eric F.) 05 November 2002 (2002-11-05)<br>See entire document. | 1-14 |
| A | JP 6349405 B2 (MORINAGA MILK INDUSTRY CO., LTD.) 27 June 2018 (2018-06-27)<br>See entire document. | 1-14 |

☐ Further documents are listed in the continuation of Box C.   ☑ See patent family annex.

| * Special categories of cited documents: | |
| --- | --- |
| "A" document defining the general state of the art which is not considered to be of particular relevance | "T" later document published after the international filing date or priority date and not in conflict with the application but cited to understand the principle or theory underlying the invention |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **07 April 2021** | **08 April 2021** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
| --- |
| **PCT/KR2020/018279** |

**Box No. II          Observations where certain claims were found unsearchable (Continuation of item 2 of first sheet)**

This international search report has not been established in respect of certain claims under Article 17(2)(a) for the following reasons:

1. ☑  Claims Nos.: **15-17**
because they relate to subject matter not required to be searched by this Authority, namely:

Claims 15-17 pertain to a method for treatment of the human body by surgery or therapy, as well as a diagnostic method (PCT Article 17(2)(a)(i) and PCT Rule 39.1(iv)).

2. ☐  Claims Nos.:
because they relate to parts of the international application that do not comply with the prescribed requirements to such an extent that no meaningful international search can be carried out, specifically:

3. ☐  Claims Nos.:
because they are dependent claims and are not drafted in accordance with the second and third sentences of Rule 6.4(a).

Form PCT/ISA/210 (continuation of first sheet) (July 2019)

<p style="text-align:center"><b>INTERNATIONAL SEARCH REPORT</b><br>Information on patent family members</p>

International application No.

**PCT/KR2020/018279**

| Patent document cited in search report | | | Publication date (day/month/year) | Patent family member(s) | | | Publication date (day/month/year) |
|---|---|---|---|---|---|---|---|
| WO | 2018-102397 | A1 | 07 June 2018 | CA | 3043768 | A1 | 07 June 2018 |
| | | | | CN | 110177544 | A | 27 August 2019 |
| | | | | EP | 3548005 | A1 | 09 October 2019 |
| | | | | EP | 3548005 | A4 | 17 June 2020 |
| | | | | JP | 2019-535839 | A | 12 December 2019 |
| | | | | US | 2018-0193270 | A1 | 12 July 2018 |
| KR | 10-2005-0024313 | A | 10 March 2005 | EP | 1531857 | A1 | 25 May 2005 |
| | | | | JP | 2005-534655 | A | 17 November 2005 |
| | | | | US | 2005-0250693 | A1 | 10 November 2005 |
| | | | | WO | 03-103705 | A1 | 18 December 2003 |
| WO | 2018-170332 | A1 | 20 September 2018 | None | | | |
| JP | 2002-537346 | A | 05 November 2002 | EP | 1162934 | A1 | 19 December 2001 |
| | | | | EP | 1162934 | A4 | 26 January 2005 |
| | | | | US | 2005-0208000 | A1 | 22 September 2005 |
| | | | | US | 2012-0263663 | A1 | 18 October 2012 |
| | | | | WO | 00-50057 | A1 | 31 August 2000 |
| JP | 6349405 | B2 | 27 June 2018 | EP | 3192518 | A1 | 19 July 2017 |
| | | | | EP | 3192518 | A4 | 28 March 2018 |
| | | | | EP | 3192518 | B1 | 20 January 2021 |
| | | | | WO | 2016-039356 | A1 | 17 March 2016 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **R. J. CANNELL ; S. J. KELLAM ; A. M. OWSIANKA ; J. M. WALKER.** *Cannell method,* 1988

<110>    University-Industry Cooperation Group of Kyung Hee University

<120>    COMPOSITION FOR ENHANCING SKIN ELASTICITY AND IMPROVING WRINKLES
         COMPRISING MILK EXOSOMES

<130>    20PP31106PCT

<150>    KR 10-2019-0176590
<151>    2019-12-27

<160>    10

<170>    KoPatentIn 3.0

<210>    1
<211>    23
<212>    DNA
<213>    Artificial Sequence

<220>
<223>    Timp1 Forward


<400>    1
accaccttat accagcgtta tga                                                23


<210>    2
<211>    21
<212>    DNA
<213>    Artificial Sequence

<220>
<223>    Timp1 Reverse


<400>    2
ggtgtagacg aaccggatgt c                                                  21


<210>    3
<211>    21
<212>    DNA
<213>    Artificial Sequence

<220>
<223>    Procollagen Forward


<400>    3
gtgcgatgac gtgatctgtg a                                                  21


<210>    4
<211>    19
<212>    DNA
<213>    Artificial Sequence

<220>
<223>    Procollagen Reverse


<400>    4

cggtggtttc ttggtcggt                                                    19


<210>      5
<211>      23
<212>      DNA
<213>      Artificial Sequence

<220>
<223>      CD34 Forward


<400>      5
ctacaacacc tagtaccctt gga                                               23


<210>      6
<211>      22
<212>      DNA
<213>      Artificial Sequence

<220>
<223>      CD34 Reverse


<400>      6
ggtgaacact gtgctgatta ca                                               22


<210>      7
<211>      19
<212>      DNA
<213>      Artificial Sequence

<220>
<223>      Elastin Forward


<400>      7
gcaggagtta agcccaagg                                                    19


<210>      8
<211>      20
<212>      DNA
<213>      Artificial Sequence

<220>
<223>      Elastin Reverse


<400>      8
tgtagggcag tccatagcca                                                   20


<210>      9
<211>      23
<212>      DNA
<213>      Artificial Sequence

<220>
<223>      MMP1 Forward

```
<400>     9
attggagcag caagaggctg gga                                              23


<210>     10
<211>     23
<212>     DNA
<213>     Artificial Sequence

<220>
<223>     MMP1 Reverse


<400>     10
ttccaggtat ttctggacta agt                                             23
```